# EUROPÄISCHE PATENTSCHRIFT

(11) **EP 0 405 618 B1**
(45) Veröffentlichungstag und Bekanntmachung des Hinweises auf die Patenterteilung: **20.10.1993**
(21) Anmeldenummer: 90115738.8
(22) Anmeldetag: 05.03.1988
(51) Int. Cl.: A61F 2/32, A61F 2/34

(54) **Hüftgelenk-Endoprothese**
Hip endoprosthesis
Endoprothése de la hanche

(30) Priorität: 07.03.1987 DE 3707428
(43) Veröffentlichungstag der Anmeldung: 02.01.1991
(62) Teilanmeldung aus: 88103472.2
(73) Patentinhaber: Künne, Hermann, Dr., D-59597 Erwitte (DE)
(72) Erfinder: Künne, Hermann, Dr., D-59597 Erwitte (DE)
(74) Vertreter: Thielking, Bodo, Dipl.-Ing.

(56) Entgegenhaltungen:
- EP-A- 0 201 442
- DE-A- 3 417 923
- DE-A- 3 528 151

## Beschreibung

Die Erfindung betrifft eine Hüftgelenk-Endoprothese nach dem Oberbegriff des Anspruchs 1.

Bei einer bekannten Hüftgelenk-Endoprothese dieser Art (DE-A-35 28 151) weist der Schaftteil einen benachbart zu der Kugel angeordneten Bereich auf, der von dem Basisteil des Schafts getrennt ausgebildet ist und mit Hilfe einer sich in Längsrichtung des Schafts erstreckenden Zugschraube in Richtung auf die Kugel bewegt werden kann. Bei einem Bewegen in dieser Richtung wird der Schaftteil mit einem vorgesehenen Kragen auf dem entsprechend abgeschnittenen Oberschenkelknochen fixiert. Bei der bekannten Endoprothese ist es nachteilig, daß deren Einbaulage durch die Anlagefläche am Knochen bestimmt ist, an der der Kragen des Schaftteils anliegt. Weiterhin erscheint es nachteilig, daß wegen der Anordnung des verschiebbaren Bereichs lediglich in unmittelbarer Nähe zur Kugel eine sichere Fixierung des Schaftteils in der Knochenhöhle nicht möglich ist. Die Fixierung beschränkt sich auf den oberen Randbereich, während das untere Schaftende nicht fixiert ist.

Ausgehend von diesem Stand der Technik liegt der Erfindung die Aufgabe zugrunde, eine Hüftgelenk-Endoprothese nach dem Oberbegriff des Anspruchs 1 so auszubilden, daß der Schaftteil in jeder gewünschten Position zum Oberschenkelknochen fixierbar ist und eine Fixierung über die gesamte Länge des Schaftteils möglich ist.

Die Lösung dieser Aufgabe erfolgt dadurch, daß der Schaftteil aus zwei sich zu einem Schaft ergänzenden Einzelbereichen und einem Schaftelement besteht, das zwischen die Einzelbereiche in Schaftlängsrichtung einführbar ist, wobei das Spreizelement aus einem Metallblatt besteht, das auf einer Breitseite eine Rippe aufweist, welche in eine Aufnahmenut des gegenüberliegenden Schaftteils eingreift, und daß die Hüftgelenk-Endoprothese einen in einer Aufnahme öffnung das Beckens fixierbaser Pfannenteil aufweist, wobei die Kugel in einer Pfannenschale abläuft, die in einer geschlitzten Hülse angeordnet ist, welche auf ihrer Oberfläche mit Abständen voneinander angeordnete, widerhakenartig wirkende Rastelemente aufweist.

Bei einer bevorzugten Ausführungsform besteht der Schaftteil aus einer Titanlegierung.

Es hat sich ferner als zweckmäßig erwiesen, daß die Vorsprünge auf der Umfangsfläche des Schaftteils raspelartig oder messerartig ausgebildete Vorsprünge sind.

Schließlich erweist es sich als besonders zweckmäßig, daß die mittels des Spreizelements auseinander spreizbaren Bereiche des Schaftteils über Schrauben miteinander verbunden sind.

Die erfindungsgemäße Hüftgelenk-Endoprothese erlaubt eine genaue Justierung und eine sichere Fixierung in der Knochenhöhle über praktisch die gesamte Einbaulänge. Dabei ist eine sichere Fixierung insbesondere auch am von der Kugel abgewandten Ende möglich. Dies hat zur Folge, daß eine besonders sichere Verbindung mit dem Oberschenkelknochen erzielt wird.

Nachstehend wird ein Ausführungsbeispiel der Erfindung anhand der Zeichnung im einzelnen beschrieben. Es zeigen:
- Figur 1: - eine Seitenansicht des Schaftteils der Prothese,
- Figur 2: - eine Seitenansicht auf das Schaftteil gemäß Figur 1 in Richtung des Pfeils II,
- Figur 3: - einen Querschnitt durch den unteren Bereich des Schaftteils ohne eingeführtes Spreizelement,
- Figur 4: - einen Schnitt durch das Schaftteil mit eingeführtem Spreizelement,
- Figur 5: - eine perspektivische Darstellung einer ersten Ausführungsform des Pfannenteils in perspektivischer Explosionsdarstellung,
- Figur 6: - eine zweite Ausführungsform des Pfannenteils in perspektivischer Explosionsdarstellung.

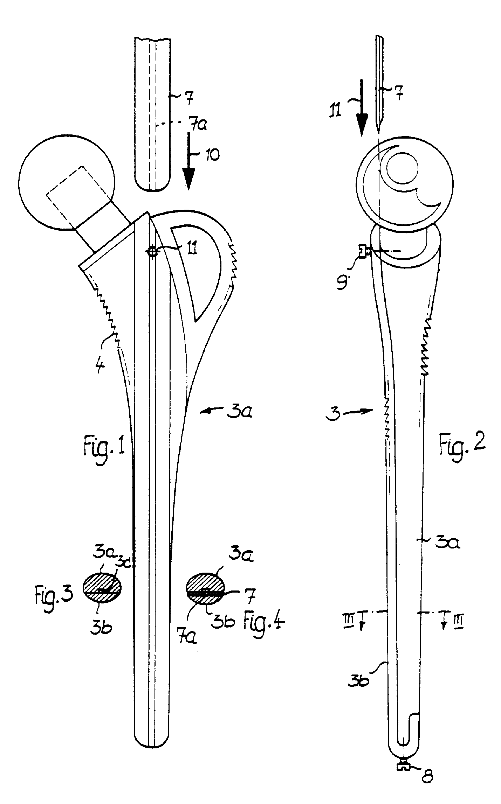

In den Figuren 1 und 2 ist der Schaffteil 3 der Hüftgelenk-Endoprothese sichtbar, wobei Figur 1 nur den Bereich 3a zeigt und im oberen Bereich ein Spreizelement 7, während Figur 2 die beiden Bereiche 3a und 3b im zusammengesetzten Zustand ohne eingeführtes Spreizelement zeigt.

Die beiden Bereiche 3a und 3b werden in der gemäß Figur 2 dargestellten Lage zusammengefügt in die Knochenhöhle des Oberschenkelknochens eingeführt. Dabei sind die beiden Bereiche 3a und 3b über die untere Schraube 8 miteinander verschraubt. Die obere Schraube 9 ist zunächst gelöst. Anschließend wird im Einsetzzustand des Schaftteils 3 das blattförmige Spreizelement 7, welches eine Rippe 7a aufweist, in Richtung des Pfeils 10 zwischen die beiden Bereiche 3a und 3b eingetrieben. Dabei gleitet die Rippe 7a in der Aufnahmenut 3c des Bereichs 3a. Wenn das Spreizelement in seiner Endposition liegt, ist dessen oberes Ende unterhalb der Schraube 9 angelangt. Danach kann die im Bereich 3b sitzende Schraube 9 in die Gewindebohrung 11 des Bereichs 3a eingeschraubt werden.

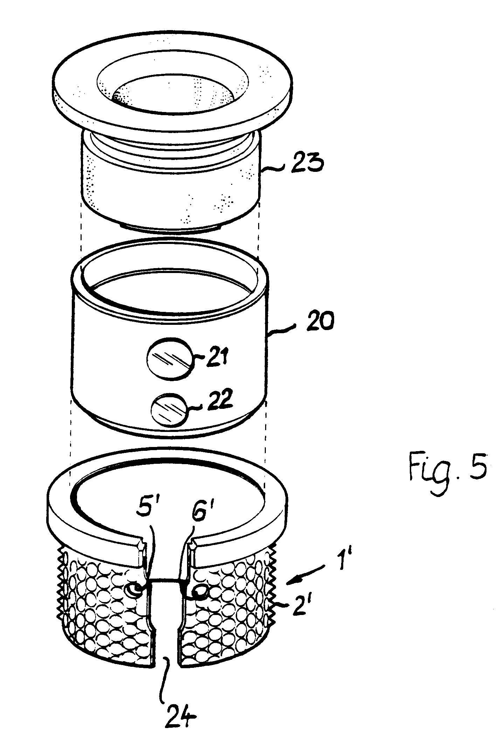

Die Vorsprünge 4 können auch andere als die dargestellte sägezahnartige Kontur haben. Sie können sich bei Bedarf über alle gewünschten Bereiche der Außenkontur des Schaftteils 3 erstrecken. Die Ausbildung und Größe der Vorsprünge kann unterschiedlich gewählt werden. Die beiden Schnittzeichnungen bei Figuren 3 und 4 sind der Einfachheit halber ohne Vorsprünge gezeichnet.

In Figur 5 ist der Pfannenteil dargestellt. Er besteht aus einem Ringkörper 1', einem metallischen Ring 20 und einer Kunststoffpfanne 23. Der Ringkörper 1' weist auf seinem Umfang raspelartig oder messerartig ausgebildete Vorsprünge 2' auf. In der Zeichnung ist nicht dargestellt, daß diese Vorsprünge, die auch eine andere Form und Orientierung aufweisen können, leicht abwärts geneigt sein können. Bei einer leichten Abwärtsneigung der messerartig ausgebildeten Vorsprünge hat das Spreizen des metallischen Ringbereichs 1' zur Folge, daß der Pfannenteil sich fest gegen den Boden der zylindrischen Aufnahmeöffnung legt und in dieser Lage gehalten wird.

Zum Einbau des Ringkörpers 1' wird die Trennfuge 24 mit einem Spezialwerkzeug, welches in die Montageöffnungen 5 und 6 eingesetzt wird, zunächst verringert und anschließend wieder vergrößert. In der gespreizten, vergrößerten Lage des Ringkörpers 1' ist die Trennfuge 24 am größten. In dieser Position haben sich die Vorsprünge 2' in die umgebende Knochenmasse eingedrückt. Ein geschlossener zylindrischer Ring 20 wird anschließend in den eingebauten Ringkörper 1' eingeschoben. Dabei liegen sich die Vorsprünge 22 und 21 an die einander gegenüberliegenden Stirnflächen der Trennfuge 24 an. Die Vorsprünge 22 und 21 und die Trennfuge 24 können so bemessen sein, daß beim Einschieben des Metallrings 20 eine endgültige und weitere Spreizung des Körpers 1' erfolgt. Im Metallring 20 sitzt die Kunststoffpfanne 23 mit einer Pfannenschale zur Aufnahme der Kugel des Schaftteils der Prothese.

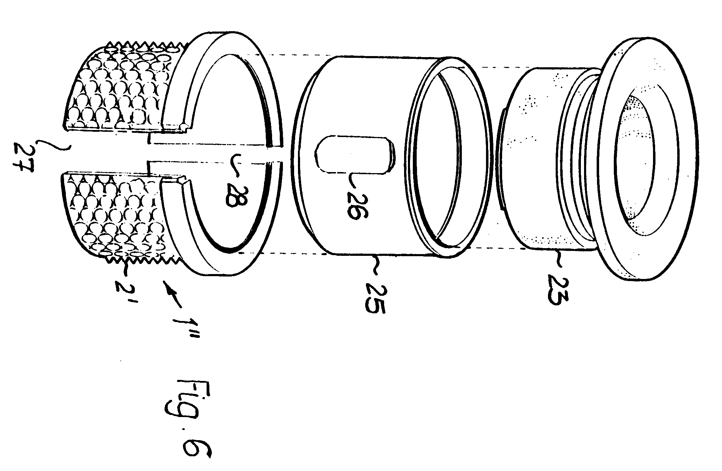

Bei der weiteren Ausführungsform gemäß Figur 6 ist ein Ringkörper 1' vorgesehen, der aus zwei sich zu einem Ring ergänzenden Hälften besteht. Die beiden Halbschalen sind entlang zweier einander gegenüberliegender Trennfugen 27 und 28 geteilt. Der Einsatz der beiden Halbschalen erfolgt mit in den Trennfugen aneinanderstoßenden Stirnflächen. Anschließend werden die beiden Halbschalen mittels eines Spezialwerkzeugs so weit wie möglich radial nach außen gepreßt In der gespreizten Lage wird der geschlossene Metallring 25 mit der Kunststoffpfanne 23 eingeschoben oder leicht eingeschlagen. Dabei legt sich der als Distanzelement dienende Vorsprung 26 an die Stirnseiten der einander gegenüberliegenden Flanken der Trennfuge 27 an. Ein entsprechender Vorsprung, der nicht dargestellt ist, kann zum Auseinanderdrücken der Flanken der gegenüberliegenden Trennfuge 28 auf der gegenüberliegenden Seite des Metallrings 25 vorgesehen sein.

## Patentansprüche

1. Hüftgelenk-Endoprothese mit einem im Oberschenkelknochen fixierbaren Schaftteil (3) mit Kugel, wobei der Schaftteil spreizbar ausgebildet ist und an seinem Umfang Vorsprünge (4) vorgesehen sind,
dadurch gekennzeichnet,
daß der Schaftteil (3) aus zwei sich zu einem Schaft ergänzenden Einzelbereichen (3a und 3b) und einem Spreizelement (7) besteht, das zwischen die Einzelbereiche in Schaftlängsrichtung einführbar ist, wobei das Spreizelement (7) aus einem Metallblatt besteht, das auf einer Breitseite eine Rippe (7a) aufweist, welche in eine Aufnahmenut (3c) des gegenüberliegenden Schaftteils (3a) eingreift, und daß die Hüftgelenk-Endoprothese einen in einer Aufnahme öffnung des Beckens fixierbaser Pfannenteil aufweist, wobei die Kugel in einer Pfannenschale abläuft, die in einer geschlitzten Hülse (1'; 1'') angeordnet ist, welche auf ihrer Oberfläche mit Abständen voneinander angeordnete, widerhakenartig wirkende Rastelemente (2') aufweist.

2. Hüftgelenk-Endoprothese nach Anspruch 1,
dadurch gekennzeichnet,
daß der Schaftteil (3) aus einer Titanlegierung besteht.

3. Hüftgelenk-Endoprothese nach Anspruch 1 oder 2,
dadurch gekennzeichnet,
daß die Vorsprünge (2) auf der Umfangsfläche des Schaftteils (3) raspelartig oder messerartig ausgebildete Vorsprünge sind.

4. Hüftgelenk-Endoprothese nach einem oder mehreren der Ansprüche 1 bis 3,
dadurch gekennzeichnet,
daß die mittels des Spreizelements (7) auseinander spreizbaren Bereiche (3a; 3b) des Schaftteils (3) über Schrauben (8; 9) miteinander verbunden sind.

## Claims

1. Hip joint endoprosthesis with a shaft part (3) with ball fixable in the femur wherein the shaft part is designed to expand and projections (4) are provided on its circumference,
characterised in that the shaft part (3) consists of two individual areas (3a and 3b) which make up one shaft, and of an expanding element (7) which can be inserted in the longitudinal direction of the shaft between the individual areas wherein the expanding element (7) is made of a metal leaf which has on a broad side a rib (7a) which engages in a socket groove (3c) of the opposite shaft part (3a) and that the hip joint endoprosthesis has a socket part fixable in a socket opening of the pelvis, wherein the ball moves in a socket dish which is mounted in a slit sleeve (1'; 1'') which has on its surface detent elements (2') which are spaced out from each other and act like barbs.

2. Hip joint endoprosthesis according to claim 1,
characterised in that the shaft part (3) is made from titanium alloy.

3. Hip joint endoprosthesis according to claim 1 or 2 characterised in that the projections (2) are formed like rasps or blades on the circumferential surface of the shaft part (3).

4. Hip joint endoprosthesis according to one or more of claims 1 to 3
characterised in that the areas (3a;3b) of the shaft part (3) which can spread out from each other by means of the expanding element (7) are connected together by screws (8;9)

## Revendications

1. Endoprothèse coxo-fémorale comprenant un corps de prothèse (3) fixable dans le fémur et pouvant être écarté, lequel est pourvu d'une tête et présente, sur sa périphérie, des saillies (4),
caractérisée par le fait
que le corps de prothèse (3) est composé de deux zones individuelles (3a et 3b), qui se complètent, et d'un élément d'écartement (7), insérable entre les deux zones individuelles, dans le sens de la longueur, lequel consiste en une feuille métallique et présente, sur son côté large, une nervure (7a) qui s'engage dans une gorge de réception (3c) de la zone (3a) opposée, et
que l'endoprothèse coxo-fémorale comporte une pièce pourvue d'une cavité cotyloïde artificielle fixable dans un orifice de réception du bassin, la tête se déplaçant dans une cavité cotyloïde artificielle, logée dans un manchon fendu (1'; 1''), dont la surface présente des éléments de crantage (2'), genre barbes, disposés à intervalle les uns des autres.

2. Endoprothèse coxo-fémorale selon revendication 1,
caractérisée par le fait
que le corps de prothèse (3) est exécuté en alliage de titan.

3. Endoprothèse coxo-fémorale selon revendication 1 ou 2,
caractérisée par le fait
que les saillies, prévues sur la surface périphérique du corps de prothèse (3), sont conçues sous forme de dents de râpe ou de lames de coupe.

4. Endoprothèse coxo-fémorale selon une ou plusieurs revendications 1 à 3,
caractérisée par le fait
que les zones (3a; 3b) du corps de prothèse (3), écartables à l'aide des éléments d'écartement (7), sont reliées ensemble par des vis (8; 9)
